# EUROPÄISCHE PATENTANMELDUNG

(11) **EP 4 616 887 A1**
(43) Veröffentlichungstag der Anmeldung: **17.09.2025**
(21) Anmeldenummer: 24163667.9
(22) Anmeldetag: 14.03.2024
(51) Int. Cl.: A61M 5/315

(54) **STOPFEN FÜR EINEN MEDIZINISCHEN SPRITZENKÖRPER SOWIE MEDIZINISCHER SPRITZENKÖRPER**

(71) Anmelder: Inductio AG, 6052 Hergiswil (CH)
(72) Erfinder: SPICHIGER, Marco, 2544 Bettlach (CH); KOLLER, Horst, 8730 Uznach (CH)
(74) Vertreter: Tergau & Walkenhorst Intellectual Property GmbH

(57) **Zusammenfassung**

Ein Stopfen (20) für einen medizinischen Spritzenkörper (2), insbesondere zur Verwendung in einer vorbefüllten Spritze (1), soll in besonderem Maße eine vorgesehene Verwendung des Spritzenkörpers (2) als Primärpackmittel ermöglichen. Dazu ist der Stopfen (20) erfindungsgemäß als mehrkomponentiger Stopfen (20) ausgeführt und umfasst als Komponenten:
- einen zentralen Stopfenkörper (30) aus einem ersten, vergleichsweise harten Kunststoff,
- einen den zentralen Stopfenkörper (30) zumindest teilweise umschließenden Dichtmantel (32) aus einem zweiten, im Vergleich zum ersten Kunststoff weicheren Kunststoff, sowie
- einen RFID-Transponder (22).

## Beschreibung

Die Erfindung bezieht sich auf einen mehrkomponentigen Stopfen für einen medizinischen Spritzenkörper, insbesondere zur Verwendung in einer medizinischen Spritze. Sie betrifft weiter einen medizinischen Spritzenkörper mit einem solchen Stopfen.

Im Spritzenkörper einer medizinischen Spritze ist üblicherweise ein in Längsrichtung verschiebbarer Kolben oder Stopfen vorgesehen, der über ein am proximalen Ende des Spritzenkörpers angeordnetes Betätigungselement, beispielsweise einen Betätigungsstößel oder auch ein Element mit automatisiertem Antrieb, verschoben werden kann. Damit kann über einen solchen Stopfen Druck auf den im Spritzenkörper vorgehaltenen Wirkstoff ausgeübt werden, so dass dieser über eine am distalen Ende des Spritzenkörpers angeordnete Hohlnadel ausgetragen und somit appliziert werden kann.

Gerade bei der Verabreichung hochwertiger oder ggf. auch für die Umgebung potentiell schädlicher Wirkstoffe ist die Dichtigkeit eines solchen Spritzensystems von besonderer Bedeutung. Dabei kann auch die Dichtwirkung des im Spritzenkörper an dessen Innenwand entlang gleitenden Stopfens von Bedeutung sein. Um hierfür eine besonders hohe Dichtigkeit zu erreichen, ist beispielsweise aus der DE 10 006 560 A1 ein Spritzenkolben oder -stopfen in zweikomponentiger Ausführung bekannt, bei dem auf einem zentralen Stopfenkörper in der Art einer äußeren Hülle ein Dichtmantel aus einem im Vergleich zum Stopfenkörper weicheren Material aufgebracht ist.

Im Hinblick auf eine erleichterte Handhabung und hohe Zuverlässigkeit gerade im Umgang mit hochwertigen Arzneimitteln oder Wirkstoffen ist es zunehmend erstrebenswert, solche, mit geeignet dichtend ausgeführten Stopfen versehene Spritzenkörper auch als Primärpackmittel beispielsweise in der Art vorbefüllter Spritzenkörper einzusetzen, die zur Verabreichung des Wirkstoffs dann mit geeigneten weiteren Komponenten, wie beispielsweise Nadel- oder Stößelsystemen zur Vervollständigung einer medizinischen Spritze, einem Antriebskopf zur Bereitstellung eines Autoinjektors oder einem Auslösemechanismus zur Bereitstellung eines Pen-Injektors, kombiniert werden können. Unter "Primärpackmittel" ist in diesem Zusammenhang insbesondere ein Verpackungs- oder Behältnissystem zu verstehen, in das der Wirkstoff oder das Arzneimittel unmittelbar nach seiner Herstellung oder Produktion eingefüllt und darin vorgehalten oder zwischengelagert werden kann, bis es zu einer Umfüllung, Verabreichung oder einerm sonstigen nachgelagerten Behandlungsschritt kommt.

Der Erfindung liegt die Aufgabe zugrunde, einen verbesserten Stopfen für einen medizinischen Spritzenkörper anzugeben, mit dem der solchermaßen ausgestattete Spritzenkörper für eine vorgesehene Verwendung als Primärpackmittel in besonderer Weise ertüchtigt wird.

Diese Aufgabe wird erfindungsgemäß gelöst mit einem mehrkomponentigen Stopfen, der als Komponenten umfasst:
- einen zentralen Trägerkörper aus einem ersten, vergleichsweise harten Kunststoff,
- einen den zentralen Trägerkörper zumindest teilweise umschließenden Dichtmantel aus einem zweiten, im Vergleich zum ersten Kunststoff weicheren Kunststoff, sowie
- einen RFID-Transponder.

Vorteilhafte Ausgestaltungen der Erfindung sind in den Unteransprüchen angegeben und ergeben sich durch Merkmale und Aspekte gemäß nachfolgender Beschreibung.

Die Erfindung geht von der Überlegung aus, dass ein hinsichtlich seiner Eigenschaften verbesserter Stopfen der genannten Art mit im Sinne der Dichtwirkung einerseits, aber auch hinsichtlich der Gleit- und Hafteigenschaften beim Verschieben innerhalb des Spritzenkörpers noch weiter verbesserten Eigenschaften ausgestattet sein sollte. Dies kann gemäß einem Aspekt der Erfindung durch die Kombination zweier Komponenten für den Stopfen, nämlich einerseits eines zentralen, formgebenden Versteifungselements aus einem vergleichsweise harten Kunststoff und andererseits aus einem dieses umgebenden, spezifisch auf eine hohe Dichtwirkung ausgelegten Dichtelement aus einem vergleichsweise weichen Kunststoff erreicht werden. Um aber darüber hinausgehend noch gezielt eine besondere Eignung für eine Verwendung des Spritzenkörpers als Primärpackmittel zu begünstigen, ist gemäß einem Aspekt der Erfindung als zusätzliche Komponente des Stopfens noch ein RFID-Transponder vorgesehen. Dabei liegt die überraschende Erkenntnis zu Grunde, dass - ausgehend von der Herstellung und Abfüllung des Wirkstoffs oder Arzneimittels - ein als vorbefüllter Spritzenkörper bereitgestellter, mit Stopfen versehener Spritzenkörper selbst bei unterschiedlichen zwischenzeitlichen Transport- oder Lagerschritten immer und durchgängig mit dem einmal abgefüllten Wirkstoff in Kontakt steht und verbunden ist. Somit eignen sich die Komponenten eines solchen Spritzenkörpers, und dabei gerade der Stopfen, in besonderem Maße als Träger für ein Informationselement, in dem wesentliche charakteristische Informationen über den abgefüllten Wirkstoff, wie beispielsweise Inhaltsstoffe, Abfülldatum, Chargennummer oder dergleichen, für eine spätere Nutzung bereitgehalten werden können.

Gemäß einem Aspekt der Erfindung ist somit der Stopfen als diejenige Komponente des Spritzenkörpers ausgewählt, die als Träger für ein solches Informationselement dienen soll. Gemäß einem Aspekt der Erfindung ist dabei als Informationselement ein RFID-Transponder, auch als "RFID-Tag" oder "Funketikett" bezeichnet, vorgesehen; es kann sich auch um ein so genanntes "RFID Label" handeln. Ein solcher RFID-Transponder ist per Funk und somit kontaktlos auslesbar, was die Handhabung und Logistik gerade bei abgefüllten Wirkstoffen oder Arzneimitteln und den damit einhergehenden möglicherweise enormen Stückzahlen enorm erleichtert. Ein RFID-System besteht nämlich in der Regel aus einem derartigen RFID-Transponder, der sich am oder im jeweiligen Gegenstand und einen kennzeichnenden Code und ggf. weitere Informationen enthält, sowie einem Lesegerät zum Auslesen dieser Kennung. RFID-Transponder können sehr klein in ihren Abmessungen sein und dementsprechend in den Stopfen integriert werden. Darüber hinaus besteht die Möglichkeit, RFID-Transponder über ein spezielles Druckverfahren stabiler Schaltungen aus Polymeren herzustellen.

Mittels der auf dem RFID-Chip oder -Transponder gespeicherten Daten soll insbesondere eine nachvollziehbare und manipulationssichere Nachverfolgung des jeweiligen Produkts ermöglicht werden. Dementsprechend können diesbezügliche, für das im jeweiligen Spritzenkörper befindliche Produkt oder den Wirkstoff charakteristische Informationen auf dem Chip oder Transponder hinterlegt sein. Alternativ oder insbesondere zusätzlich kann gemäß einem Aspekt der Erfindung aber auch vorgesehen sein, mittels der hinterlegten Daten den einzelnen Spritzenkörper eindeutig identifizieren zu können. Dazu könnte beispielsweise eine Chargennummer oder dergleichen, vorzugsweise in Kombination mit einer Materialnummer, zur Identifikation hinterlegt sein. Bevorzugt und gemäß einem Aspekt der Erfindung ist daher zusätzlich oder alternativ zu einer solchen Chargennummer eine individuelle, für den einzelnen Spritzenkörper charakteristische Kennung hinterlegt.

In vorteilhafter Ausgestaltung umfasst der RFID-Transponder einen RFID-Chip und eine mit diesem verbundene RFID-Antenne. Für eine besonders kompakte Bauweise des Stopfens ist der RFID-Transponder in vorteilhafter Ausgestaltung am zentralen Trägerkörper angeordnet.

Zur Bereitstellung einer hohen Abdichtwirkung auch bei besonders guten Gleit- und Reibeieigenschaften bei einer Verschiebung im Spritzenkörper sollte insbesondere die Haft- oder Gleitreibung des Stopfens an der Innenwand des Spritzenkörpers besonders gering gehalten werden, um eine vergleichsweise sanfte und schonende Bewegung innerhalb des Spritzenkörpers mit gering gehaltenen Losbrechkräften und dergleichen zu ermöglichen. Gemäß einem Aspekt der vorliegenden Erfindung kann dies erreicht werden durch eine gezielte Nutzung der Verformbarkeit des Dichtmantels aufgrund seiner Materialeigenschaften eines im Vergleich zum zentralen Stopfenkörper weicheren Materials. Diese kann mittels einer geeigneten Formgebung des Dichtmantels besonders wirksam genutzt werden, wobei durch ein geeignetes Außenprofil geeignete Verformungszonen bereitgestellt werden können. Dazu kann gemäß einem Aspekt der Erfindung der Dichtmantel einen im Wesentlichen zylindrischen Außenmantel aufweisen, der mit einer Mehrzahl von in Richtung der Zylinderachse gesehen beabstandet zueinander positionierten umlaufenden Dichtrippen versehen ist.

Vorteilhafterweise ist zudem die Breite der oder jeder Dichtrippe des Dichtmantels geringer gewählt als der Abstand zwischen zwei benachbarten Dichtrippen. In alternativer oder zusätzlicher vorteilhafter Weiterbildung weist der Dichtmantel in seinem endseitigen Bereich einen im Vergleich zum Außendurchmesser des zentralen Stopfenkörpers vergrößerten Innendurchmesser auf, so dass sich im montierten Zustand eine endseitig umlaufende Nut zwischen dem zentralen Stopfenkörper und dem Dichtmantel ergibt. Sowohl diese Nut als auch die Raumbereich zwischen benachbarten Dichtrippen können dann als Verformungszonen für die Dichtlippen selbst dienen, so dass deren Anpresskraft an die Innenwand des Spritzenkörpers geringgehalten werden kann.

Bevorzugt und in als eigenständig erfinderisch angesehener Ausgestaltung ist ein medizinischer Spritzenkörper mit einem Stopfen der beschriebenen Art ausgestattet; in dem Spritzenkörper ist somit gemäß einem Aspekt der Erfindung ein mehrkomponentiger Stopfen der genannten Art geführt.

Der so ausgestaltete Spritzenkörper ist gemäß weiteren Aspekten der Erfindung Teil oder Komponente einer vorbefüllten medizinischen Spritze, eines Pen-Injektors oder eines Auto-Injektors. Mit anderen Worten wird als eigenständig erfinderisch angesehen die Verwendung eines Stopfens der vorstehend beschriebenen Art in einem medizinischen Spritzenkörper, in einer vorbefüllten medizinischen Spritze, in einem Pen-Injektor oder in einem Auto-Injektor.

Ein Ausführungsbeispiel der Erfindung wird anhand einer Zeichnung näher erläutert. Darin zeigen:
- Fig. 1: eine medizinische Spritze in perspektivischer Ansicht,
- Fig. 2: die Spritze nach Fig. 1 mit entfernter Nadelschutzkappe in perspektivischer Ansicht,
- Fig. 3: einen Spritzenkörper der Spritze nach FIG. 1 im Längsschnitt,
- Fig. 4: einen alternativen Spritzenkörper im Längsschnitt,
- Fig. 5: eine erste Variante eines im Spritzenkörper nach Fig. 3 oder 4 geführten Stopfen im Längsschnitt,
- Fig. 6: eine zweite Variante eines im Spritzenkörper nach Fig. 3 oder 4 geführten Stopfen im Längsschnitt,
- Fig. 7: den Stopfen nach Fig. 6 in perspektivischer Ansicht,
- Fig. 8: den Stopfen nach Fig. 6 in seitlicher Ansicht, und
- Fig. 9: den Stopfen nach Fig. 6 in rückseitiger perspektivischer Ansicht.

Gleiche Teile sind in allen Figuren mit denselben Bezugszeichen versehen.

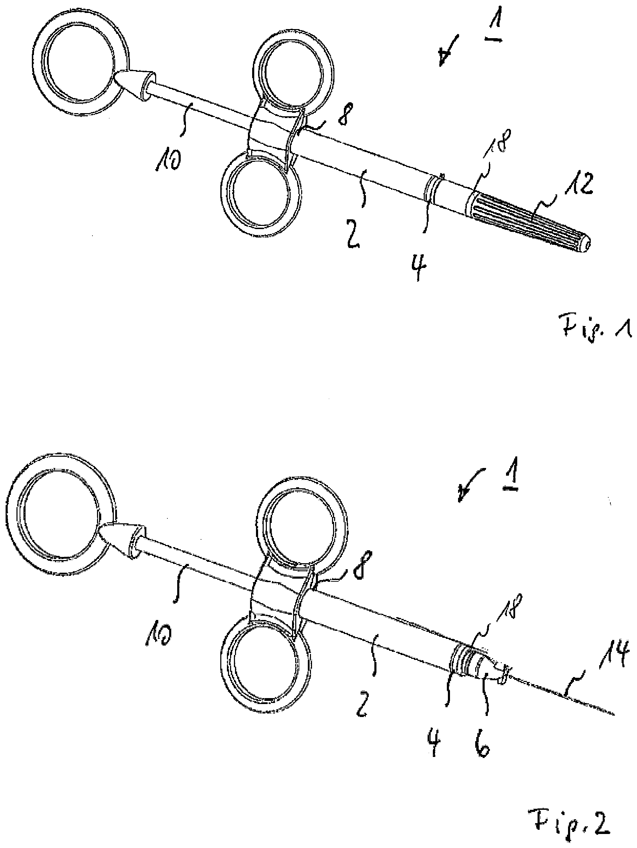

Die medizinische Spritze 1 gemäß Fig. 1 umfasst im Wesentlichen drei Baugruppen, nämlich einen den medizinischen Spritzenkörper 2 bildenden Wirkstoffbehälter (auch als Kartuschen- oder Patroneneinheit bezeichnet), der einerseits an seinem distalen Ende 4 mit einem Nadelkopf 6 oder Nadelhalter als zweite Baugruppe und andererseits an seinem proximalen Ende 8 mit einem Betätigungselement 10 als dritte Baugruppe verbunden ist. In Fig. 1 ist die Spritze 1 zudem mit am Nadelkopf 6 angebrachter Nadelschutzkappe 12 gezeigt. Fig. 2 zeigt die Spritze 1 hingegen in einsatzfähigem Zustand mit entfernter Nadelschutzkappe 12, so dass die im Nadelkopf 6 angebrachte Hohlnadel 14 freiliegt.

Im Ausführungsbeispiel ist als Betätigungselement 10 ein Griffstück gezeigt, wie es beispielsweise in einer zahnärztlichen Behandlung bei der Anästhesie zum Einsatz kommen könnte. Es umfasst im gezeigten Beispiel ein mit Fingerringen versehenes Basiselement, in dem ein endseitig ebenfalls mit einer Fingerlasche versehener Betätigungsstößel in seiner Längsrichtung verschiebbar geführt ist. Alternativ kann das Betätigungselement 10 aber auch als gewöhnliches Spritzen-Griffstück oder aber auch als automatisiertes Betätigungselement 10 in der Art eines Autoinjektors oder in der Art eines Pen-Injektors ausgeführt sein. Die Verbindung des Betätigungselements 10 mit dem Spritzenkörper 2 kann in unterschiedlichen Variationen ausgeführt sein, beispielsweise als Schraub-, Steck- oder Klickverbindung. Der Spritzenkörper 2 ist entsprechend einer herkömmlichen Bauweise zylindrisch oder rohrförmig als ein Spritzengehäuse bildender Hohlkörper 16 ausgeführt, der zur Aufnahme des medizinischen Wirkstoffs vorgesehen ist. Der Nadelkopf 6 oder Nadelhalter kann am vorderen oder distalen Ende 4 des Hohlkörpers 16 angeschraubt oder auch aufgesteckt werden, je nachdem, welche Bauweise für die Verbindung gewählt ist.

Gemäß einem Aspekt der Erfindung kann die Nadelschutzkappe 12 als Originalitätsverschluss ausgeführt sein. Dazu umfasst sie den eigentlichen Kappenkörper, an den in der Art eines Siegels abreißbar ein Sicherungsring 18 angeformt ist. Beim Öffnen der Spritze, also beim Entfernen der Nadelschutzkappe 12 vom Nadelhalter 6, wird der Sicherungsring 18 abgerissen, und er verbleibt auf dem Nadelhalter 6. Damit wird für den Benutzer eindeutig erkennbar, dass die Spritze 1 bereits benutzt und der Wirkstoffcontainer "angebrochen" wurde.

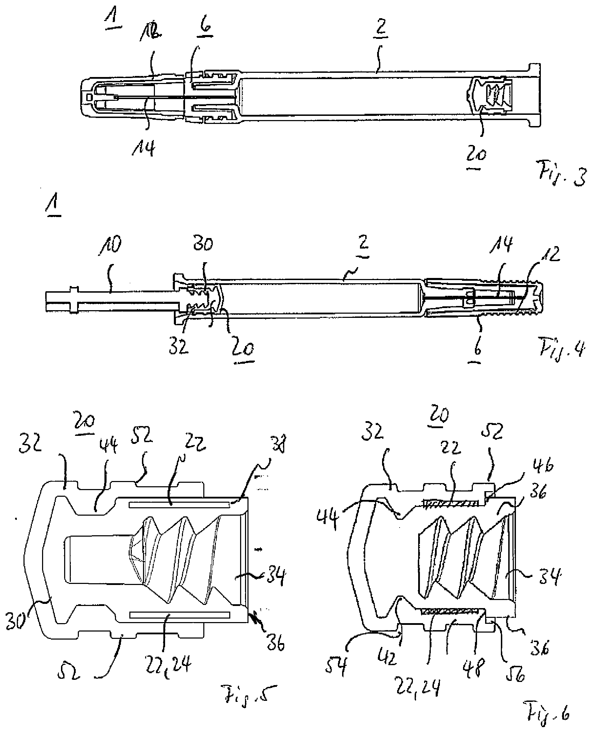

Der mit der angebrachten Nadelschutzkappe 12 versehene Spritzenkörper 2 ist in Fig. 3 im Längsschnitt gezeigt. In der dargestellten Variante ist der Nadelhalter 6 mittels beispielsweise einer Luer-Verbindung am distalen Ende 4 an den Spritzenkörper 2 angeschraubt; alternativ könnte hierfür aber auch eine Steck-, Klemm- oder Rastverbindung, eine Verbindung über einen Bajonettverschluss oder eine anderweitig geeignete Verbindung vorgesehen sein. Beispielhaft ist der Spritzenkörper in Fig. 4 in einer Variante für eine Klemmverbindung mit dem Nadelkopf 6 gezeigt, in diesem Fall mit eingebrachtem Betätigungselement 10.

Innerhalb des den Spritzenkörper 2 bildenden Hohlkörpers 16 ist in an sich durchaus üblicher Ausgestaltung ein in Längsrichtung verschiebbarer Kolben oder Stopfen 20 vorgesehen. Der Stopfen 20 ist dabei in an sich durchaus üblicher Ausgestaltung über das jeweils am proximalen Ende 8 des Spritzenkörpers 2 angeordnete Betätigungselement 10, beispielsweise einen Betätigungsstößel oder auch ein Element mit automatisiertem Antrieb, innerhalb des Hohlkörpers 16 verschiebbar, so dass beispielsweise der im Hohlkörper 16 vorgehaltene Wirkstoff über die Hohlnadel 14 ausgetragen und somit appliziert werden kann. Der Stopfen 20 ist dabei derart dimensioniert, dass er dichtend an der Innenwand des Hohlkörpers 16 anliegt; im Längsbereich des Innenraums des Hohlkörpers 16 zwischen dem distalen Ende 4 und dem Stopfen 20 wird somit das Reservoir für den Wirkstoff ausgebildet, in dem dieser vorgehalten werden kann. Bei angebrachtem Nadelkopf 6, bei dem die Hohlnadel 14 mit ihrem proximalen Ende in dieses Reservoirvolumen hineinragt, kann der Stopfen 20 innerhalb des Hohlkörpers 16 in Richtung zum distalen Ende 4 hin verschoben werden, so dass der Wirkstoff durch die Hohlnadel 14 aus dem Innenraum herausgedrückt wird.

Der Spritzenkörper 2 und insbesondere der für diesen vorgesehene Stopfen 20 ist gemäß einem Aspekt der Erfindung für eine besonders gute Eignung für eine Verwendung als Primärpackmittel ausgelegt. Dabei soll im Hinblick auf eine erleichterte Handhabung und hohe Zuverlässigkeit auch im Umgang mit hochwertigen Arzneimitteln oder Wirkstoffen die Nutzung des Spitzenkörpers 2 möglichst von der Abfüllung ab, d. h. nach einer Befüllung mit Wirkstoff unmittelbar nach dessen Herstellung, über eine vorübergehende oder ggf. auch länger andauernde (Zwischen-) Lagerung bis hin zur Verabreichung ermöglicht werden. Hierfür ist der Stopfen 20 im Sinne eines verbesserten Stopfens geeignet ausgelegt, so dass der medizinische Spritzenkörper 2 für eine vorgesehene Verwendung als Primärpackmittel in besonderer Weise ertüchtigt wird.

Gemäß einem Aspekt der Erfindung wird dies mit dem Stopfen 20 erreicht, indem der Stopfen 20 einerseits für besonders hohe Dichtigkeitsanforderungen und auch gute Gleiteigenschaften ausgelegt ist. Andererseits wird gemäß einem Aspekt der Erfindung aber auch der Erkenntnis Rechnung getragen, dass gerade bei einer solchen Ertüchtigung als Primärpackmittel der Stopfen 20 als eine der wenigen, wenn nicht die einzige mit Ausnahme der Innenwand des Hohlkörpers 16, Komponenten angesehen werden kann, die von der Herstellung und Abfüllung an bis hin zur Verabreichung des Wirkstoffs permanent mit diesem in direktem Kontakt steht. Diese Erkenntnis kann nach einem Aspekt der Erfindung gezielt genutzt werden, indem der Stopfen 20 als Mittel zur fälschungs- und manipulationssicheren Etikettierung oder Kennzeichnung des in den Spritzenkörper 2 abgefüllten Wirkstoffs ertüchtigt wird.

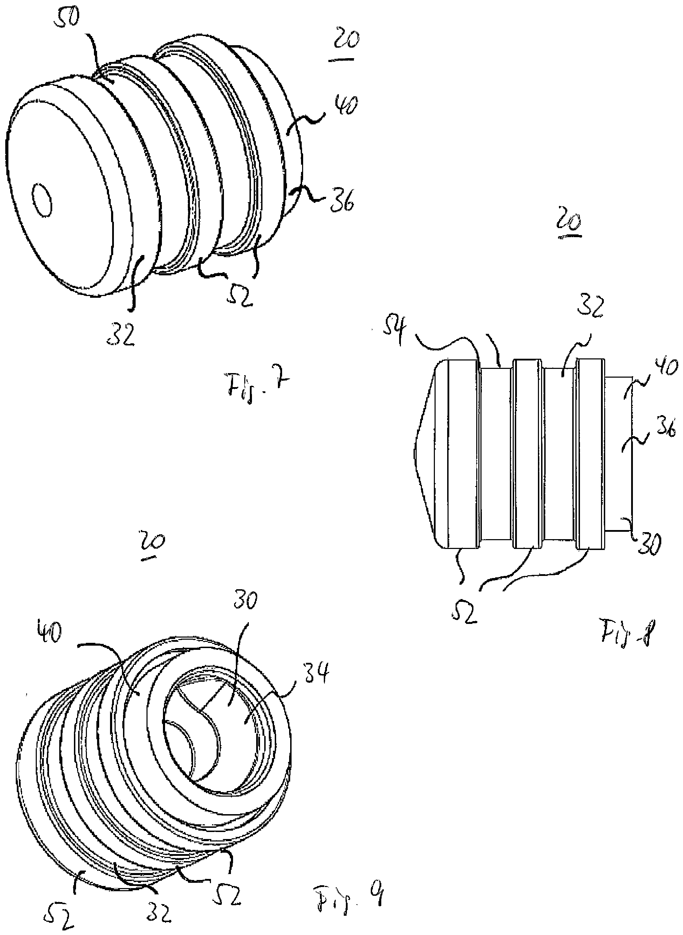

Um in Anbetracht dieser Auslegungsziele einen auf besonders einfache Weise herstellbaren Stopfen 20 anzugeben, ist der Stopfen 20 gemäß einem Aspekt der Erfindung als mehrkomponentiger, insbesondere so genannter "2K-Stopfen" ausgeführt und mit einem integrierten RFID-Transponder 22 versehen. Details zur Ausgestaltung des Stopfens 20 sind in zwei Varianten den Darstellungen im Längsschnitt in Fig. 5, 6, in perspektivischer Ansicht in Fig. 7, in seitlicher Ansicht in Fig. 8 und in weiterer perspektivischer Ansicht "von hinten" in Fig. 9 entnehmbar.

Gemäß einem Aspekt der Erfindung ist dient der RFID-Transponder 22, der auch als "RFID-Tag" oder "Funketikett" bezeichnet werden kann, somit als Informationselement, in dem wichtige, für den Behälterinhalt charakteristische Informationen hinterlegt und für eine spätere Nutzung bereitgestellt werden können. Gemäß einem Aspekt der Erfindung ist im RFID-Transponder 22 des Stopfens 20 insbesondere eine individuelle, für den einzelnen Spritzenkörper charakteristische Kennung hinterlegt. Der solchermaßen ausgelegte RFID-Transponder 22 ist später per Funk und somit kontaktlos auslesbar, was die Handhabung und Logistik gerade bei abgefüllten Wirkstoffen oder Arzneimitteln und den damit einhergehenden möglicherweise enormen Stückzahlen enorm erleichtert. Damit kann ein solchermaßen ausgerüsteter Spritzenkörper 2 und der darin befindliche Wirkstoff auf besonders einfache Weise nachverfolgt ("getrackt") und geeignet überwacht werden, wobei dies auch vollständig automatisiert erfolgen kann.

Gemäß einem Aspekt der Erfindung umfasst der RFID-Transponder 22 einen RFID-Chip 24 und eine mit diesem verbundene RFID-Antenne, die beispielsweise gedruckt sein kann.

Der gemäß einem Aspekt der Erfindung zweikomponentig oder als "2K-Stopfen" ausgeführte Stopfen 20 umfasst einen zentralen Stopfenkörper 30 aus einem vergleichsweise harten Kunststoff, im Ausführungsbeispiel und gemäß einem Aspekt der Erfindung aus Polypropylen (PP), der von einem aus im Vergleich dazu weicheren Kunststoffmaterial, im Ausführungsbeispiel und gemäß einem Aspekt der Erfindung TPE, gebildeten Dichtmantel 32 umgeben ist. Der zentrale Stopfenkörper 30 dient dabei im Wesentlichen zur Form- und Strukturstabilität und zur Weiterleitung der eingeleiteten Kräfte, insbesondere in Längsrichtung bei der Verschiebung des Stopfens 20 innerhalb des Spritzenkörpers 2. Dazu kann der zentrale Stopfenkörper 30 innenseitig mit einem Aufnahmekanal 34, beispielsweise mit einem Gewinde, versehen sein, über den eine Verbindung mit einem externen Betätigungsmittel, beispielsweise dem Betätigungselement 10 oder dem Betätigungsstößel der Spritze 1, ermöglicht ist. In als synergistisch angesehener Weise kann gemäß einem Aspekt der Erfindung der RFID-Transponder 22 für eine besonders kompakte Bauweise des Stopfens 20 und für eine zuverlässige und sichere Lagerung am zentralen Stopfen- oder Trägerkörper 30 angeordnet sein. Damit kann eine Beeinträchtigung der Dichtwirkung des Stopfens 20 durch den eingebrachten Transponder 22 minimiert oder sogar ganz verhindert werden.

Durch die als eigenständig erfinderisch angesehene Ausführung des Stopfens 20 als 2K-Stopfen mit einer Kombination des zentralen Stopfenkörpers 30 mit dem vergleichsweise weichen Dichtmantel 32 kann mittels geeigneter Geometrie- und Parameterwahl (beispielsweise genaue Materialzusammensetzung oder Auswahl des verwendeten Kunststoffs) dieser Komponenten einerseits die Anpresskraft des Dichtmantels 32 an die Innenwand des Hohlkörpers 16 und somit die Dichtigkeit des Systems ebenso gut und genau eingestellt werden wie die Gleitreibungskräfte beim Verschieben des Stopfens 20 im Hohlkörper 16. Damit kann ein solchermaßen aufgebauter Stopfen 20 als synergistisches Element in Zusammenwirkung beispielsweise mit einer zugeordneten Antriebseinheit für das Betätigungselement 10 im Sinne einer kontrollierten Vorgabe der Systemeigenschaften wie beispielsweise Losbrechkräfte und dergleichen genutzt werden. Des Weiteren kann durch die Kombination dieser Komponenten die üblicherweise bei der Verwendung reiner TPE-Stopfen bei langer Lagerzeit auftretende Schrumpfung und damit ein Dichtigkeitsverlust vermieden werden. Ein solcher 2K-Stopfen 20 ermöglicht somit unter anderem auch zuverlässig vergleichsweise lange Lagerzeiten vorbefüllter Spritzen.

Diese gemäß einem Aspekt der Erfindung vorgesehene Bauweise, also die Bereitstellung des inneren, vergleichsweise härteren Stopfenkörpers 30 mit einer Ummantelung durch den vergleichsweise weicheren Dichtmantel 32, mit dem im Aufnahmekanal 34 vorgesehen stabilen Gewinde wird gerade in Kombination mit dem integrierten RFID-Transponder 22 als besonders vorteilhaft angesehen. Durch diese Bauweise kann der Stopfenkörper 30 und mit diesem der Stopfen 20 insgesamt fest mit einem Betätigungselement 10 der Spritze 1 verbunden werden. Dadurch können axiale Verschiebungen des Stopfens 20 innerhalb des Hohlkörpers 16 sowohl in "Vorwärts- " als auch in "Rückwärtsrichtung" erfolgen, d. h. es können wahl- und bedarfsweise Schub- oder auch Zugwirkungen auf den Stopfen 20 ausgeübt werden. Damit sind einerseits Anwendungen wie z. B. Aspirationstests und/oder Rekonstitutionen von Medikamenten in einer solchen Spritze 1 ermöglicht. Andererseits können auch vergleichsweise hohe Haltekräfte in einem solchen System toleriert werden, die mit herkömmlichen Stopfenverbindungen nicht möglich wären. Des Weiteren ist üblicherweise beim Einschrauben der Kolbenstange in ein entsprechendes Gewinde eines reinen TPE- oder Gummistopfens ein erhöhter Anpressdruck in Kauf zu nehmen, so dass sich die Haft- oder Gleitreibung entsprechend erhöht. Dieser Nachteil ist durch die gemäß einem Aspekt der Erfindung nunmehr vorgesehene Ausgestaltung des Stopfens 20 nicht mehr in Kauf zu nehmen.

Der Dichtmantel 32 kann den zentralen Stopfenkörper 30 gemäß Aspekten der Erfindung in Längsrichtung gesehen teilweise oder auch nahezu vollständig umgeben. In Fig. 5 ist der Stopfen 20 in einer Ausgestaltung gezeigt, bei der lediglich eine teilweise Abdeckung in Längsrichtung vorgesehen ist. Dies führt dazu, dass in einem proximalen Bereich 36 der Stopfenkörper 30 frei liegt und somit eine Montagefläche 38 für eine Anbringung des RFID-Transponders 22 bietet. Demgegenüber zeigt Fig. 6 eine Ausgestaltung des Stopfens 20, bei der der zentrale Stopfenkörper 30 in Längsrichtung nahezu vollständig vom Dichtmantel 32 umschlossen ist. In dieser Ausführungsform kann die Montagefläche 38 "innenliegend", also im vom Dichtmantel 32 umschlossenen Teilbereich des zentralen Stopfenkörpers 30, vorgesehen sein, so dass sich eine Art gekapselte Anbringung des RFID-Transponders 22 zwischen dem zentralen Stopfenkörper 30 und dem Dichtmantel 32 vornehmen lässt. Eine solche gekapselte Anbringung kann je nach erwarteter Beanspruchung des Stopfens 20 sehr vorteilhaft sein.

In der in Fig.6 gezeigten Ausführungsform bleibt somit lediglich im proximalen Anschlussbereich 36 des zentralen Stopfenkörpers 30 ein endseitiger Flansch 40 vom Dichtmantel 32 unbedeckt. Der zentrale Stopfenkörper 30 ist in beiden Ausführungsformen für eine formschlüssige Fixierung des Dichtmantels 32 am zentralen Stopfenkörper 30 mit einer umlaufenden Haltenut 42 versehen, in die eine Innenlippe 44 des Dichtmantels 32 eingreift. Des Weiteren weist der zentrale Stopfenkörper 30 im Bereich des endseitigen Flanschs 40 eine umlaufende Kante 46 auf, auf der eine korrespondierende Innenkante 48 des Dichtmantels 32 aufsitzt.

In einer als eigenständig erfinderisch angesehenen Ausführung ist der Stopfen 20 für eine besonders hohe Dichtwirkung innerhalb des jeweiligen Spritzenkörpers 2 ausgelegt. Dabei wird gemäß einem Aspekt der Erfindung gezielt die relativ gute Verformbarkeit des vergleichsweise weichen Dichtmantels 32 mit einer geeigneten Geometriewahl und Formgebung kombiniert. Dazu weist der Dichtmantel 32 einen im Wesentlichen zylindrischen Außenmantel 50 auf, der mit einer Mehrzahl von in Richtung der Zylinderachse gesehen beabstandet zueinander positionierten umlaufenden Dichtrippen 52 versehen ist. Im Bereich der Dichtrippen 52 kann der Stopfen 20 gemäß einem Aspekt der Erfindung mit einem gewissen Übermaß ausgeführt sein, d. h. einen geringfügig größeren Durchmesser aufweisen als der Innendurchmesser des jeweiligen Spritzenkörpers 2. Aufgrund der Verformbarkeit des den Dichtmantel 32 bildenden Kunststoffs kann dieser sich somit an die Innenkontur des Spritzenkörpers 2 anpassen, wobei aufgrund der Formgebung und der vorgesehenen beabstandet voneinander angeordneten Dichtrippen 52 Ausweichräume bereitgestellt sind, in die die verformten Dichtrippen 52 ausweichen können. Somit ist es möglich, einerseits auf zuverlässige Weise eine hohe Dichtwirkung zu erzielen, wobei andererseits die auftretenden Haft- und Gleitreibungskräfte infolge des Kontakts mit der Innenwand des jeweiligen Spritzenkörpers 2 ausreichend gering gehalten werden können. Gemäß einem Aspekt der Erfindung ist dabei die Breite der oder jeder Dichtrippe 52 geringer gewählt als der Abstand zwischen zwei benachbarten Dichtrippen 52. Durch diese Ausgestaltung des Stopfens 20 kann somit bei hoher Dichtigkeit eine besonders gute und störungsfreie Verschiebbarkeit des Stopfens 20 im jeweiligen Spritzengehäuse 2 erreicht werden.

Zusätzlich begünstigt werden diese Effekte durch die gemäß einem Aspekt der Erfindung vorgesehene Positionierung und Ausgestaltung der Haltenut 42 mit korrespondierender Innenlippe 44, die im Schnitt gesehen jeweils eine Art V-förmige Kontur aufweisen. Damit wird unter Nutzung der Verformbarkeit des relativ weichen Materials des Dichtmantels 32 eine Verformungszone 54 geschaffen, in die hinein Material aus der in Vorschubrichtung des Stopfens 20 gesehen vordersten Dichtrippe 52 ausweichen kann. Damit wird der Reibwiderstand des im Spritzenkörper 2 gleitenden Stopfens 20 gemäß einem Aspekt der Erfindung noch weiter verringert.

Zur weiteren Begünstigung des Reibverhaltens des Stopfens 20 weist gemäß einem Aspekt der Erfindung der Dichtmantel 32 im Bereich des endseitigen Flanschs 40 und seiner Innenkante 48 einen im Vergleich zum Außendurch-messer des zentralen Stopfenkörpers 30 geringfügig vergrößerten Innendurch-messer auf, so dass sich im montierten Zustand endseitig ein Spalt in der Art einer umlaufenden Nut 56 zwischen diesen Komponenten ergibt. Durch diese umlaufende Nut 56 wird eine weitere Verformungszone bereitgestellt, in die bei einer Reibbelastung Material der in Vorschubrichtung des Stopfens 20 gesehen letzten Dichtrippe 52 ausweichen kann.

Gemäß einem Aspekt der Erfindung ist der Stopfen 20 durch ein 2K-Spritzgussverfahren hergestellt.

Vorstehend wurde der Stopfen 20 in den Ausführungsbeispielen für den Einsatz in einem Spritzenkörper 2 in einer vorbefüllten Spritze 1 beschrieben. Selbstverständlich sind gemäß Aspekten der Erfindung aber auch andere Anwendungsbereiche möglich und auch als erfindungsgemäß angesehen, beispielsweise in einem Pen-Injektor oder einem Auto-Injektor.

### Bezugszeichenliste

- 1: Medizinische Spritze
- 2: Spritzenkörper
- 4: distales Ende
- 6: Nadelkopf
- 8: proximales Ende
- 10: Betätigungselement
- 12: Nadelschutzkappe
- 14: Hohlnadel
- 16: Hohlkörper
- 18: Sicherungsring
- 20: Stopfen
- 22: RFID-Transponder
- 30: Stopfenkörper
- 32: Dichtmantel
- 34: Aufnahmekanal
- 36: proximaler Bereich
- 38: Montagefläche
- 40: Flansch
- 42: Haltenut
- 44: Innenlippe
- 46: Kante
- 48: Innenkante
- 50: Außenmantel
- 52: Dichtrippe
- 54: Verformungszone
- 56: Nut

## Patentansprüche

1. Stopfen (20) für einen medizinischen Spritzenkörper (2), der als mehrkomponentiger Stopfen (20) ausgeführt ist und als Komponenten umfasst:
- einen zentralen Stopfenkörper (30) aus einem ersten, vergleichsweise harten Kunststoff,
- einen den zentralen Stopfenkörper (30) zumindest teilweise umschließenden Dichtmantel (32) aus einem zweiten, im Vergleich zum ersten Kunststoff weicheren Kunststoff, sowie
- einen RFID-Transponder (22).

2. Stopfen (20) nach Anspruch 1, in dessen RFID-Transponder (22) ein individueller, für den einzelnen Spritzenkörper charakteristischer Kennwert hinterlegt ist.

3. Stopfen (20) nach Anspruch 1 oder 2, bei dem der RFID-Transponder (22) am zentralen Stopfenkörper (30) angeordnet ist.

4. Stopfen (20) nach einem der Ansprüche 1 bis 3, dessen RFID-Transponder (22) einen RFID-Chip (24) und eine mit diesem verbundene RFID-Antenne umfasst.

5. Stopfen (20) nach einem der Ansprüche 1 bis 4, dessen Dichtmantel (32) einen im Wesentlichen zylindrischen Außenmantel (50) aufweist, der mit einer Mehrzahl von in Richtung der Zylinderachse gesehen beabstandet zueinander positionierten umlaufenden Dichtrippen (52) versehen ist.

6. Stopfen (20) nach Anspruch 5, bei dem die Breite der oder jeder Dichtrippe (52) geringer gewählt ist als der Abstand zwischen zwei benachbarten Dichtrippen (52).

7. Medizinischer Spritzenkörper (2), in dem ein mehrkomponentiger Stopfen (20) nach einem der Ansprüche 1 bis 6 geführt ist.

8. Vorbefüllte medizinische Spritze (1) mit einem Spritzenkörper (2) nach Anspruch 7.

9. Pen-Injektor mit einem Spritzenkörper nach Anspruch 7.

10. Auto-Injektor mit einem Spritzenkörper nach Anspruch 7.
